## Europäisches Patentamt

## European Patent Office

## Office européen des brevets

(19)

(11) Veröffentlichungsnummer: **0 106 946**
**B1**

(12) **EUROPÄISCHE PATENTSCHRIFT**

(45) Veröffentlichungstag der Patentschrift:
**15.10.86**

(51) Int. Cl.⁴: **A 61 F 2/30**

(21) Anmeldenummer: **83107331.7**

(22) Anmeldetag: **26.07.83**

(54) Gelenkendoprothese.

(30) Priorität: **15.10.82 CH 6019/82**

(43) Veröffentlichungstag der Anmeldung:
**02.05.84 Patentblatt 84/18**

(45) Bekanntmachung des Hinweises auf die Patenterteilung:
**15.10.86 Patentblatt 86/42**

(84) Benannte Vertragsstaaten:
**AT DE FR IT**

(56) Entgegenhaltungen:
**CH-A-507 704**
**DE-A-2 548 077**
**FR-A-2 179 166**
**GB-A-720 092**
**US-A-4 005 495**

(73) Patentinhaber: **GEBRÜDER SULZER AKTIENGESELLSCHAFT, Zürcherstrasse 9, CH-8401 Winterthur (CH)**

(72) Erfinder: **Frey, Otto, Walrütistrasse 56, CH- 8404 Winterthur (CH)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf (DE)**

LIBER, STOCKHOLM 1986

## Beschreibung

Die Erfindung betrifft eine Gelenkendoprothese, insbesondere Femurkopfprothese, bestehend aus einem in den Knochen einsetzbaren Verankerungsschaft, der an seinem freien Ende einen konischen Zapfen (Vaterkonus) mit einer seine Verformbarkeit erhöhenden Oberflächenstruktur trägt, auf dem eine mit einer entsprechenden Aussparung (Mutterkonus) versehene Gelenkkugel aufsteckbar ist.

Eine vielfach verwendete Massnahme, um die Gelenkkugel einer Endoprothese, insbesondere eine Femurkopfprothese, mit dem Verankerungsschaft zu verbinden, besteht in einer Konussteckverbindung, bei der die Gelenkkugel über einen, gegebenenfalls selbsthemmenden, Konus auf einen eine Oberflächenstruktur aufweisenden Zapfen eines Verankerungsschaftes aufgesteckt ist (DE-A- 25 48 077). Besonders bei relativ grossen Gelenkkugeln mit erhöhten Torsionsmomenten, wie sie für sogenannte Frakturprothesen verwendet werden, kommt es nach der Implantation häufig zu unerwünschten Relativdrehungen zwischen Gelenkkugel und Zapfen.

Weiterhin ist bekannt (CH-A- 507 704; DE-C- 22 20 304), über eine ähnliche konische Steckverbindung eine Gelenkkugel auf einem Zapfen zu befestigen, bei der die Zapfenachse nicht mit der Symmetrieachse der Gelenkkugel zusammenfällt, um die Stellung der Gelenkkugel relativ zur Femurachse an die individuellen Unterschiede im Skelettbau der verschiedenen Patienten annähern zu können. Bei derartigen Prothesen mit "schiefer" Stellung der beiden Achsen muss gewährleistet sein, dass sich die Gelenkkugel nicht relativ zum Zapfen während der "Lebenszeit" der Prothese verdrehen kann. Daher ist es bekannt, am Fuss des Zapfens als Rippen, Nasen, Zähne oder ähnliches ausgebildete Verdrehsicherungen die in entsprechende radiale Nuten im Mantel des Mutterkonus eingreifen (CH-A-507 704), oder an der Stirnfläche der Gelenkkugel Nocken, die in Nuten in der Stirnfläche des Verankerungsschaftes eingreifen (DE-C-2 220 304), vorzusehen. Ganz abgesehen, dass die Herstellung der Nuten im Mutterkonus einen hohen zusätzlichen Aufwand erfordert, haben diese Verdrehsicherungen den Nachteil, dass sie keine stufenlose Einstellung der Gelenkkugel relativ zum Zapfen erlauben, sondern nur eine der Anzahl der Nuten entsprechende relative Drehung und Fixierung der Kugel gegenüber dem Zapfen ermöglichen.

Es ist weiterhin eine kappenartige Femurkopfprothese bekannt (US-A-4.005.495), die einen Mutterkonus enthält, der direkt auf den Knochen des entsprechend vorbereiteten natürlichen Femurkopfes aufgesetzt wird. Der Mutterkonus enthält unter anderem Vorsprünge oder Erhebungen, die das Gewebewachstum und das Anwachsen von Gewebe fördern sollen und gleichzeitig als primäre Befestigung und als Verdrehsicherung dienen. Die Prothesenkappe wird zusätzlich durch eine Druckplatte gehalten, für die eine Ausfräsung im Knochen vorgesehen ist. Wegen der Druckplatte ist der Mutterkonus der Prothesenkappe nicht rotationssymmetrisch ausgebildet; die Prothesenkappe lässt sich daher nur in einer bestimmten vorgegebenen Lage auf dem Knochen fixieren. Die in ihrem Querschnitt halbkreisförmigen Erhebungen oder Vorsprünge sind darüberhinaus nicht geeignet, Mikrobewegungen zwischen Prothese und Knochen zu verhindern.

Aufgabe der Erfindung ist es, eine Verdrehsicherung zu schaffen, mit der die Gelenkkugel vom Operateur ohne Beachtung der bestimmten Winkelstellung zwischen Zapfen und Gelenkkugel auf dem Zapfen befestigt werden kann, und die darüberhinaus stufenlos einstellbar gegenüber dem Zapfen des Verankerungszapfen ist. Mit der vorliegenden Erfindung wird diese Aufgabe dadurch gelöst, dass als Verdrehsicherung mindestens eine Nase am verjüngten Ende des Mutterkonus angeordnet ist; unter einer "Nase" wird dabei - in Anlehnung an die Umgangssprache - ein aus der Konusfläche des Mutterkonus herauswachsender Vorsprung mit steilen Seitenflanken verstanden, dessen Höhe mit Fortschreiten seiner Länge zunimmt; eine einfache Art der Verwirklichung besteht im vorliegenden Fall beispielsweise darin, den Nasenrücken als Hohlkonus mit einem vom Mutterkonus unterschiedlichen, nämlich grösseren, Konuswinkel auszubilden.

Beim Aufsetzen und Einschlagen oder -pressen der Gelenkkugel dringt die in den Hohlraum des Mutterkonus vorspringende Nase in die verformbare Oberfläche des Zapfens ein und bewirkt so die gewünschte Verdrehsicherung; da die möglichen Scherkräfte, die eine Verdrehung der Kugel bewirken könnten, relativ klein sind, genügt im Prinzip eine einzige derartige Nase zur Lösung der gestellten Aufgabe. Selbstverständlich kann man jedoch auch mehrere Nasen über den Umfang des Mutterkonus verteilt vorsehen; in Verbindung mit dem aus dem Mutterkonus herauswachsenden Nasenrücken ermöglichen mehrere - auf dem Umfang verteilte-Nasen eine selbsttätige Zentrierung des Gelenkkopfes relativ zum Zapfen beim Einschlagen.

Da der Vaterkonus auf seinem ganzen Umfang eine gleichmässige Strukturierung ohne bevorzugte Bereiche aufweist, kann die Gelenkkugel in jeder beliebigen Stellung der Nase relativ zum Umfang des Vaterkonus aufgesetzt und befestigt werden, womit die beiden Forderungen, dass der Operateur nicht auf eine bestimmte Stellung achten muss, und dass bei "schiefen" Achsen eine stufenlose Einstellbarkeit vorhanden ist, in einfacher Weise zu erfüllen sind.

Wie vorstehend beschrieben, soll die Nase des Mutterkonus in die Struktur der Oberfläche des Vaterkonus eindringen; selbstverständlich darf der dafür notwendige Kraftaufwand nicht übermässig gross sein, um Beschädigungen an Kugel oder Zapfen zu vermeiden. Es ist daher vorteilhaft, wenn die maximale Höhe der Nase 0,5 mm und ihre maximale Breite 3 mm betragen.

Die neue Verdrehsicherung ist bei allen für derartige Prothesen üblichen Werkstoffen anwendbar, sofern das Material für die Gelenkkugel schwerer verformbar ist als die Oberflächenbereiche des Zapfens. Selbstverständlich kann die Verformbarkeit der Oberflächen des Vaterkonus auch gegebenenfalls durch die Auswahl eines weicheren Materials für den Zapfen erzeugt werden.

Im folgenden wird die Erfindung anhand eines Ausführungsbeispiels im Zusammenhang mit der Zeichnung näher erläutert.

Fig. 1 ist ein Längsschnitt durch eine auf einen Zapfen aufgesteckte Gelenkkugel;

Fig. 2 gibt den Schnitt II-II von Fig. 1 wieder, während die

Fig. 3 - 5, stark vergrössert, verschiedenartig strukturierte Oberflächenbereiche des Zapfens im Schnitt zeigen.

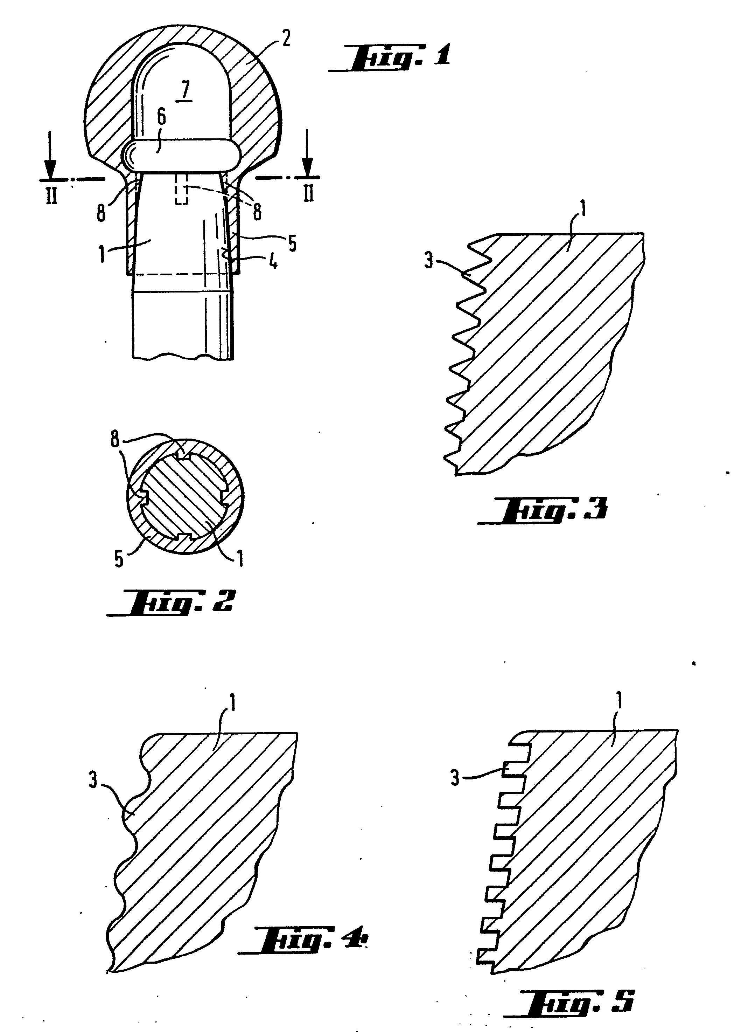

Auf einen Zapfen 1 eines nicht weiter dargestellten Verankerungsschaftes einer Femurkopfprothese ist die Gelenkkugel 2 über eine selbsthemmende, konische Steckverbindung befestigt. Die Mantelfläche des den Vaterkonus bildenden Zapfens 1 ist mit einer Strukturierung 3 versehen, durch die die Verformbarkeit dieser Mantelfläche erhöht wird. Wie die Fig. 3 - 5 zeigen, bestehen derartige Strukturierungen beispielsweise aus einem Gewinde (Fig. 3), wellenförmig abgerundeten Rillen (Fig. 4) oder aus in Umfangsrichtung verlaufenden Rippen (Fig. 5). Vorteilhafterweise kann die Strukturierung 3 so ausgebildet sein - beispielsweise durch eine abnehmende Tiefe der die Struktur verursachenden Einschnitte oder Ausnehmungen -, dass die Verformbarkeit vom freien Ende des Zapfens 1 aus nach "innen" abnimmt, so dass der Zapfen 1 einer Verformung einen umso grösseren Widerstand entgegensetzt, je weiter die Gelenkkugel 2 auf ihn aufgeschoben wird.

Der Mutterkonus 4, der zwar auf den Vaterkonus abgestimmt ist, jedoch nicht notwendigerweise mit ihm übereinstimmen muss, befindet sich - bei dem gezeigten Beispiel - in einem mit der Gelenkkugel 2 aus einem Stück gefertigten, halsartigen Ansatz 5. An den Mutterkonus 4 schliesst sich in das Innere der Gelenkkugel 2 hinein eine Hinterschneidung 6 an, die ihrerseits in einen Hohlraum 7 übergeht. Die Hinterschneidung 6 ist zur Herstellung des Mutterkonus 4 erforderlich, während der Hohlraum 7 zur Gewichtsersparnis dient oder - bei gegossenen Gelenkkugeln 2 - aus giesstechnischen Gründen notwendig ist.

Erfindungsgemäss sind am verjüngten Ende des Mutterkonus 4 über den Umfang verteilt vier vorstehende Nasen 8 vorgesehen, die beim Aufpressen oder Einschlagen auf den Zapfen 1 in dessen Oberflächenstruktur 3 eindringen und so eine Sicherung gegen relative Verdrehungen der Gelenkkugel 2 gegen den Zapfen 1 bewirken.

**Patentansprüche**

1. Gelenkendoprothese, insbesondere Femurkopfprothese, bestehend aus einem in den Knochen einsetzbaren Verankerungsschaft, der an seinem freien Ende einen als Vaterkonus bezeichneten konischen Zapfen (1) mit einer seine Verformbarkeit erhöhenden Oberflächenstruktur (3) trägt, auf dem eine mit einer entsprechenden als Mutterkonus (4) bezeichneten, Aussparung versehene Gelenkkugel (2) aufsteckbar ist, dadurch <u>gekennzeichnet</u>, dass als Verdrehsicherung mindestens eine Nase (8) am verjüngten Ende des Mutterkonus (4) angeordnet ist.

2. Gelenkendoprothese nach Anspruch 1, dadurch gekennzeichnet, dass die maximale Höhe der Nase (8) 0,5 mm und ihre maximale Breite 3 mm betragen.

**Claims**

1. An endoprosthesis for an articulation, more particularly a prosthesis for a femoral head, comprising an anchorage stem which is introducible into the bone and which has at its free end a conical peg (1) called the father cone and having a surface structure (3) increasing its deformability, a ball (2) of a joint, such ball being formed with a corresponding recess called the mother cone (4), being pushable on to the peg (1), characterised in that at least one projection (8) is disposed at the narrowed end of the mother cone (4) to secure against rotation.

2. A prosthesis according to claim 1, characterised in that the maximum height of the projection (8) is 0.5 mm and its maximum width is 3 mm.

**Revendications**

1. Endoprothèse articulée, en particulier prothèse pour tête de fémur, consistant en un tige d'ancrage qui peut être insérée dans l'os et porte, à son extrémité libre, un tenon conique (1) qualifié de cône mâle ayant une structure superficielle (3) qui accroît sa déformabilité, et sur lequel peut être emboîtée une rotule d'articulation (2) dotée d'un évidement correspondant qualifié de cône femelle (4), caractérisée par le fait qu'au moins un mentonnet (8) est disposé, en tant que système anti-rotation, à l'extrémité rétréci du cône femellle (4).

2. Endoprothèse articulée selon la revendication 1, caractérisée par le fait que la hauteur maximale du mentonnet (8) est de 0,5 mm et sa largeur maximale est de 3 mm.

Fig: 1

Fig: 2

Fig: 3

Fig. 4

Fig. 5